# EUROPEAN PATENT APPLICATION

(11) **EP 4 473 961 A1**
(43) Date of publication of application: **11.12.2024**
(21) Application number: 23178367.1
(22) Date of filing: 09.06.2023
(51) Int. Cl.: A61K 9/20, A61K 31/137, A61K 31/485

(54) **ORAL SOLID PHARMACEUTICAL FORMULATION COMPRISING METHADONE/LEVOMETHADONE AND OPTIONALLY NALOXONE**

(71) Applicant: L. MOLTENI & C. DEI FRATELLI ALITTI SOCIETA' DI ESERCIZIO S.p.A., 50018 Scandicci (FI) (IT)
(72) Inventor: ANGELI, Roberto, 50018 SCANDICCI (IT); CRESCENZA, Angela, 50018 SCANDICCI (IT)
(74) Representative: Valenza, Silvia

(57) **Abstract**

The present invention describes an immediate release oral solid pharmaceutical formulation consisting of all water-soluble components and comprising methadone (Met) or levomethadone (Lev) and optionally naloxone in weight ratio Met/Nal 40-60:1 or Lev/Nal 20-30:1; the solid formulation being critical excipients free and totally dissolving in water. The solid oral formulation of the invention in presence of naloxone is effective equally as methadone/levomethdone alone in the maintenance treatment of opioid addicts and is diversion and misuse deterrent by virtue of the presence of naloxone.

## Description

### FIELD OF THE INVENTION

The present invention attains to the field of pharmaceutical oral formulations for use in the treatment of opiate addiction, particularly in attain to a pharmaceutical oral formulation containing methadone (Met) or levomethadone (Lev) and optionally naloxone (Nal).

### STATE OF THE ART

Methadone is marketed in most of the world as a racemic mixture containing two enantiomers in a 50:50 ratio; in some European countries the pure R-isoform Levomethadone is available. Levomethadone can be used at 50% of the racemic preparation with the same therapeutic efficacy.

Oral methadone (Met) or levomethadone (Lev) treatment has demonstrated positive health outcomes in opiate dependent patients however intravenous methadone is indistinguishable from intravenous heroin or other morphine-like drugs and indeed some addicts may prefer injections of methadone to heroin. Diversion of methadone supplies to the black market and misuse (usually by injection) of prescribed medication can result in adverse outcomes and is of particular concern when the medication is not administered under supervision. Daily supervised methadone maintenance minimises risks of diversion and misuse but is however expensive and associated with high rigidity that may be a deterrent to treatment for some patients. Diverted methadone is often injected with concomitant health risks and has accounted for over half of methadone overdose deaths. On the other hand, takeaway doses are an important feature of opioid substitution therapy as they allow patients to develop activities outside of medication taking. Increased takeaway doses have been associated superior retention in treatment.

Measures taken to deter diversion and misuse of methadone take-home doses have included dispensing methadone in liquid syrup form.

Unfortunately, the attractive appearance or odor of methadone take-home syrup has led to ingestion by children, often with fatal results.

The addition of naloxone (Nal) to methadone has previously been proposed to discourage the injection of takeaway doses. Naloxone is an opioid antagonist which is inactive when taken orally. Consequently, a dose of Nal could be selected that would not alter the effects of Met when a mixture of the two is administered orally; however, if the same is diversely administered parenterally, the Nal would antagonize the narcotic effects of Met, and in addition would precipitate abstinence in subjects who were physically dependent on Met or other morphine-like drugs.

Parwatikar et al. (Clin.Pharm.&Ther. 1973, 14(6), 941-948) and Nutt et al. (Clin.Pharm.&Ther. 1974, 15(2), 156-166) found that parenteral/intravenous saline Met+Nal in weight ration 10:1 antagonize methadone narcotic effects, whereas orally as a syrup the combination Met+Nal 10:1 is indistinguishable from oral methadone alone.

Loimer et al. (J.Sub.Abuse Treat. 1991, 8, 157-160) describes oral and intravenous administration of an injectable solution of Met+Nal 50:1 finding that the oral administration did not lead to any detectable withdrawal signs or symptoms whereas the intravenous administration caused a severe withdrawal syndrome.

Bell et al. (Exp.&Clin.Psycopharm. 2009, 17(3), 146-153) ascertained
the safety, tolerability, pharmacokinetics, and pharmacodynamics of oral syrup solution of Met+Nal in a 50:1 ratio compared with methadone on 10 stable methadone-maintained subjects; and
investigated the effectiveness of intramuscularly injected Met+Nal 50:1 (injectable solution) in precipitating withdrawal on 5 stable methadone-maintained subjects.

Oral solid formulations comprising methadone or levomethadone are commercially available and may include microcrystalline cellulose and/or, maize starch and/or magnesium stearate as excipients.

The European Medicine Agency (EMA) Coordination Group for Mutual Recognition and Decentralised Procedures - Human (CMDh) during a meeting of January 2022 discussed the impact of misuse of opioid substitution treatment (OST) products related to intra-venous injection of oral products containing insoluble critical excipients which can cause serious side effects, when injected i.v.. The issue was in particular related to methadone and levomethadone based oral solid formulation comprising the insoluble excipients like e.g. microcrystalline cellulose and/or maize starch and/or magnesium stearate, which when by misuse administered intravenously may cause potentially fatal sepsis, thrombosis or excipient lung disease.

Aim of this invention is to provide an oral solid formulation of methadone or levomethadone which is free of critical excipients, diversion deterrent and safe take-home for use in maintenance treatment of opioid addicts.

### DEFINITION AND ABBREVIATIONS

Met: Methadone
Lev: Levomethadone
Nal: Naloxone
PVP. polyvinylpyrrolidone

### SUMMARY OF THE INVENTION

Subject-matter of the present invention is an immediate release oral solid pharmaceutical formulation consisting of all water soluble components and comprising methadone (Met) or levomethadone (Lev) and optionally naloxone respectively in weight ratio Met/Nal 40-60:1, or Lev/Nal 20-30:1; the solid formulation being free from insoluble critical excipients and totally dissolving in water.

The solid oral formulation of the invention in presence of naloxone is effective equally as methadone/levomethdone alone in the maintenance treatment of opioid addicts and is diversion and misuse deterrent by virtue of the presence of naloxone. The oral solid formulation of the invention is notably also anyway safe in case of misuse or diversion because is completely soluble and is free of any critical excipient like microcrystalline cellulose and/or, maize starch and/or magnesium stearate which could cause potentially fatal sepsis, thrombosis or excipient lung disease.

### DETAILED DESCRIPTION OF THE INVENTION

According to the invention methadone/levomethadone and naloxone can be as free bases or as pharmaceutically acceptable salts thereof, preferably as hydrochloride salt.

According to the invention a critical excipient is a water-insoluble excipient which can cause serious or fatal side effects when injected i.v..

The solid formulation of the invention is composed by all water-soluble components and it dissolves completely in 1-3 mL of purified water at a temperature of 25-75°C with or without stirring within a time variable from 2 minutes to 5 hours depending on volume, temperature and stirring.

The solid formulation of the present invention is preferably a swallowable tablet.

The excipients of the solid formulation of the invention include at least a binder, at least a filler and at least a lubricant; all the excipients are soluble in aqueous solvent. Preferably the at least a binder is selected in the group consisting of lactose, xylitol, maize maltodextrin and mixture thereof. According to one embodiment the solid formulation comprises lactose as binder; according to another embodiment the solid formulation is lactose free and comprises xylitol and maize maltodextrin as binder. Preferably said at least a filler is mannitol.

Preferably said at least a lubricant is selected in the group consisting of poloxamer (e.g. poloxamer 188 micro).

These preferred excipients were selected after a burdensome experimentation wherein isomalt, maltitol, Citric acid monohydrate, Sodium Bicarbonate, Sodium starch glycolate, Magnesium stearate, PVP, Croscarmellose sodium, sodium starch glycolate, Sodium Lauryl Sulphate, L-leucin were tested but with unsatisfactory results.

Preferably the solid formulation of the invention contains 5, 10, 20 or 40 mg of methadone or 2.5, 5, 10 or 20 mg of levomethadone.

Preferably the solid formulation of the invention, when containing naloxone, is prepared by serial dilution of naloxone in the filler (preferably mannitol), blending with the remainder components (i.e. methadone/levomethadone, remainder filler, binder and lubricant), and direct compression.

For preparing a formulation of the invention containing Naloxone, preferably Naloxone HCl, is gradually mixed with increasing amounts of filler in order to distribute it homogeneously within the filler; then Methadone/levo-methadone and final portions of filler are added to the blend and mixed. To the mixture containing the APIs the binder, anhydrous lactose or xylitol and maltodextrin, is added.

Thereafter the lubricant is added. The obtained final blending is then subjected to direct compression.

For preparing a formulation of the invention without Naloxone, all components of the formulation, except for the lubricant, are mixed; successively the lubricant is added and then the blend is subjected to direct compression.

Preferably according to the process of the invention all the components are firstly sieved, preferably through a 20 mesh net, to remove powder agglomerates and then blended.

Preferably the solid formulation of the invention is consisting of:

| component | % w/w |
|---|---|
| Methadone HCl | 4.0-10.0 |
| Naloxone HCl | 0.1-0.2 |
| Mannitol | 45-60 |
| Lactose | 30-40 |
| Poloxamer 188 | 3.0-5.0 |

or

| component | % w/w |
|---|---|
| levomethadone HCl | 2.0-5.0 |
| Naloxone HCl | 0.1-0.2 |
| Mannitol | 47-62 |
| Lactose | 30-40 |
| Poloxamer 188 | 3.0-5.0 |

or

| component | % w/w |
|---|---|
| Methadone HCl | 4.0-10.0 |
| Naloxone HCl | 0.1-0.2 |
| Mannitol | 60.0-70.0 |
| Maltodextrin | 7.0-13.0 |
| xylitol | 5.0-10.0 |
| Poloxamer 188 | 7.0-13.0 |

or

| component | % w/w |
|---|---|
| levomethadone HCl | 2.0-5.0 |
| Naloxone HCl | 0.1-0.2 |
| Mannitol | 65.0-75.0 |
| Maltodextrin | 7.0-13.0 |
| xylitol | 5.0-10.0 |
| Poloxamer 188 | 5.0-7.0 |

or

| component | % w/w |
|---|---|
| Methadone HCl | 4.0-10.0 |
| Mannitol | 45-60 |
| Lactose | 30-40 |
| Poloxamer 188 | 3.0-5.0 |

or

| component | % w/w |
|---|---|
| levomethadone HCl | 2.0-5.0 |
| Mannitol | 47-62 |
| Lactose | 30-40 |
| Poloxamer 188 | 3.0-5.0 |

or

| component | % w/w |
|---|---|
| Methadone HCl | 4.0-10.0 |
| Mannitol | 60.0-70.0 |
| Maltodextrin | 7.0-13.0 |
| xylitol | 5.0-10.0 |
| Poloxamer 188 | 7.0-13.0 |

or

| component | % w/w |
|---|---|
| levomethadone HCl | 2.0-5.0 |
| Mannitol | 65.0-75.0 |
| Maltodextrin | 7.0-13.0 |
| xylitol | 5.0-10.0 |
| Poloxamer 188 | 5.0-7.0 |

Naloxone HCl if present in the formulation of the invention is preferably in micronized form (d90<30µm).

To avoid sticking problems of the tablet of the invention preferred tablet punchs are:
Round Ø 7mm R7 for a 5 mg Met or 2.5 mg Lev dosage
Round Ø 8.5mm R6.5 for a 10 mg Met or 5 mg Lev dosage
Round Ø 8.5mm R6.5 for a 20 mg Met or 10 mg Lev dosage
Round Ø 9.5mm R9.5 for a 40 mg Met or 20 mg Lev dosage

Preferably the solid formulation of the invention is packed in a child-proof packaging, for example in blister with a child-proof film.

The present invention can be better understood by means of the following embodiments.

### BRIEF DESCRIPTION OF THE FIGURES

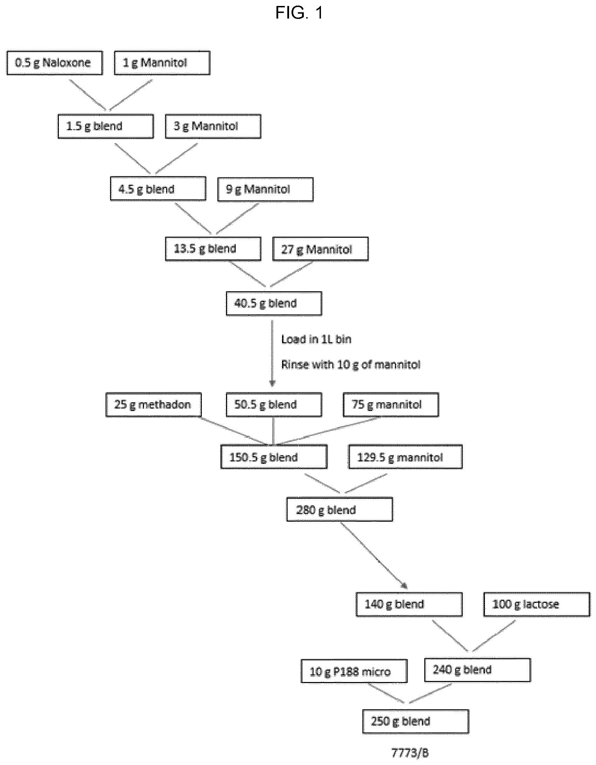

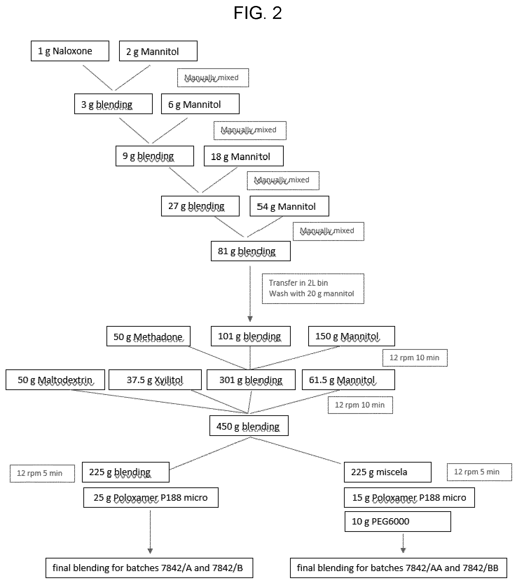

Fig. 1-2 show the serial dilution scheme for the manufacturing of a tablet according to the invention

### EXPERIMENTAL SECTION

### EXAMPLE 1 - Preparation of a tablet according to the invention containing naloxone

Tablets according to the invention were manufactured by direct compression (batch 7773/B and 7842/A), with a tablet target weight of 200mg/tablet, corresponding to a dosage of 10mg/0.2mg; the powder blend was obtained by serial dilution method (see fig. 1-2).

Naloxone HCl, preferably in micronized form d90<30µm, was gradually mixed with increasing amounts of mannitol in order to distribute it homogeneously in the diluent; then Methadone and final portions of mannitol were added to the blend and mixed. To the mixture containing the APIs anhydrous lactose or xylitol and maltodextrin (for lactose-free tablets) were added as binder.

Poloxamer P188 micro was used as lubricant. A final blending of 250g was obtained and subjected to direct compression. The tablets having Hardness (n=5): 108 N and Thickness (n=20): 4.33 mm were obtained using biconvex round punch 8.5 mm.

The formulation, corresponding to APIs dosage of 10mg/0.2mg, and the formulations for the remaining dosages are reported in the following tables.

For the lowest dosage of 5.0/0.1 mg/tab the composition reflects the percentage of the 10mg/0.2mg/tab dosage; while in the formulation for the higher dosages, 20.0/0.4 mg/tab and 40.0/0.8 mg/tab, the concentration of the APIs is increased compensated by a reduction of mannitol, the percentage of lactose/xylitol+maltodextrin and poloxamer are kept unmodified compared to batch 7773/B and 7842/A.

**Table 1 Composition of the formulation of the invention (lower dosages) (batch 7773/B)**

| **Components** | **% w/w** | **mg/tab** | **mg/tab** |
|---|---|---|---|
| Mannitol 200 SD | 50.9 | 50.9 | 101.8 |
| Methadone HCl | 5.0 | 5.0 | 10.0 |
| Naloxone HCl | 0.1 | 0.1 | 0.2 |
| Lactose anhydrous | 40.0 | 40.0 | 80.0 |
| Poloxamer P188 micro | 4.0 | 4.0 | 8.0 |
| TOT | 100 | 100 | 200 |

**Table 1 Composition of the formulation of the invention (higher dosages) (batch 7773/A)**

| **Components** | **% w/w** | **mg/tab** | **mg/tab** |
|---|---|---|---|
| Mannitol 200 SD | 45.8 | 91.6 | 183.2 |
| Methadone HCl | 10.0 | 20.0 | 40.0 |
| Naloxone HCl | 0.2 | 0.4 | 0.8 |
| Lactose anhydrous | 40.0 | 80.0 | 160.0 |
| Poloxamer P188 micro | 4.0 | 8.0 | 16.0 |
| TOT | 100 | 200 | 400 |

**Table 3 Composition of the formulation of the invention (lower dosages) (batch 7842/A)**

| **Components** | **% w/w** | **mg/tablet** | **mg/tablet** |
|---|---|---|---|
| Mannitol | 67.4 | 67.4 | 134.8 |
| Methadone HCl | 5.0 | 5.0 | 10.0 |
| Naloxone HCl | 0.1 | 0.1 | 0.2 |
| Maltodextrin | 10.0 | 10.0 | 20.0 |
| Xylitol | 7.5 | 7.5 | 15.0 |
| Poloxamer P188 micro | 10.0 | 10.0 | 20.0 |
| TOT | 100 | 100.0 | 200.0 |

**Table 4 Composition of the formulation of the invention (higher dosages) (batch 7842/B)**

| **Components** | **% w/w** | **mg/tablet*** | **mg/tablet**** |
|---|---|---|---|
| Mannitol | 62.3 | 124.6 | 249.2 |
| Methadone HCl | 10.0 | 20.0 | 40.0 |
| Naloxone HCl | 0.2 | 0.4 | 0.8 |
| Maltodextrin | 10.0 | 20.0 | 40.0 |
| Xylitol | 7.5 | 15.0 | 30.0 |
| Poloxamer P188 micro | 10.0 | 20.0 | 40.0 |
| TOT | 100 | 200.0 | 400.0 |

### Suggested tablet punch

Round Ø 7mm R7 for a 5 mg Met or 2.5 mg Lev dosage
Round Ø 8.5mm R6.5 for a 10 mg Met or 5 mg Lev dosage
Round Ø 8.5mm R6.5 for a 20 mg Met or 10 mg Lev dosage
Round Ø 9.5mm R9.5 for a 40 mg Met or 20 mg Lev dosage

The obtained tablet with lactose as binder has been subjected to dissolution test as shown in table 5

**Table 5 Preliminary dissolution results -batch 7773/B**

| **Condition** | **Visual dissolution time** | **Visual aspect** |
|---|---|---|
| 20 mL PW 37°C under stirring | ∼ 3 min | Clear transparent solution |
| 50 mL PW 37°C under stirring | ∼ 3 min | Clear transparent solution |
| 5 mL PW 50°C no stirring | ∼ 4 min | Clear transparent solution |
| 10 mL PW 50°C no stirring | ∼ 6 min | Clear transparent solution |
| 5 mL PW 70°C no stirring | ∼ 5 min | Clear transparent solution |
| 10 mL PW 70°C no stirring | ∼ 5 min | Clear transparent solution |

**Table 6 - results of dissolution tests in static conditions on low dosage lactose-free tablets (batch 7842/A) and high dosage lactose-free tablets (batch 7842/B)**

| Temperature (°C) | volume (ml) | time | batch |
|---|---|---|---|
| 75 | 1 | 13 min | 7842/A |
| 75 | 2 | 10 min | 7842/A |
| 25 | 1 | 90 min | 7842/A |
| 25 | 2 | 60 min | 7842/A |
| 75 | 2 | 20 min | 7842/B |
| 75 | 3 | 18 min | 7842/B |
| 25 | 2 | 5 h 10 min | 7842/B |
| 25 | 3 | 5 h | 7842/B |

**Table 7 - results of dissolution tests in dynamic conditions under stirring at 210 rpm on low dosage lactose-free tablets (batch 7842/A) and high dosage lactose-free tablets (batch 7842/B)**

| Volume (ml) | Temperature (°C) | Time | batch |
|---|---|---|---|
| 1 | 75 | 7 min | 7842/A |
| 2 | 75 | 5-7 min | 7842/A |
| 2 | 75 | 3 min | 7842/B |
| 3 | 75 | 2-3 min | 7842/B |
| 1 | 25 | 13-16 | 7842/A |
| 2 | 25 | 13-14 | 7842/A |
| 2 | 25 | 18 | 7842/B |
| 3 | 25 | 13 | 7842/B |

Tablets of the invention containing levomethadone and naloxone can be prepared as above by compensating the lower API amount by mannitol.

### EXAMPLE 2 - Preparation of a tablet according to the invention without naloxone

Tablets of the invention without naloxone can be obtained by direct compression as above described without the need of serial dilution and compensating the naloxone absence by mannitol.

**Table 8 - Formulations for lower dosages Methadone tablets**

| | | | **Batch 7859/A** | **Batch 7859/B** |
|---|---|---|---|---|
| | **Components** | **% w/w** | **mg/tablet** | **mg/tablet** |
| **Filler** | Mannitol | 67.5 | 67.5 | 135.0 |
| **API** | Methadone HCl | 5.0 | 5.0 | 10.0 |
| **Binder** | Maltodextrin | 10.0 | 10.0 | 20.0 |
| **Binder** | Xylitol | 7.5 | 7.5 | 15.0 |
| **Lubricant** | Poloxamer P188 micro | 10.0 | 10.0 | 20.0 |
| | TOT | 100 | 100.0 | 200.0 |

**Table 9 - Formulations for higher dosages Methadone tablets**

| | | | **Batch 7860/A** | **Batch 7860/B** |
|---|---|---|---|---|
| | **Components** | **% w/w** | **mg/tablet** | **mg/tablet** |
| **Filler** | Mannitol | 62.3 | 125.0 | 250.0 |
| **API** | Methadone HCl | 10.0 | 20.0 | 40.0 |
| **Binder** | Maltodextrin | 10.0 | 20.0 | 40.0 |
| **Binder** | Xylitol | 7.5 | 15.0 | 30.0 |
| **Lubricant** | Poloxamer P188 micro | 10.0 | 20.0 | 40.0 |
| | TOT | 100 | 200.0 | 400.0 |

For both powder blending 7859 and 7860, all components of the batch formula, except for the lubricant, were firstly sieved through 20 mesh net to remove powder agglomerates and then mixed in a stainless-steel cubic bin for 20 minutes at 12 rpm; successively, the lubricant was added and further mixed for 5 minutes at 12 rpm. The powder blending was then divided proportionally to be tabletted by direct compression in two different dosage forms each, corresponding to the following theoretical number of tablets: 7000 Methadone 5 mg tablets, 4000 Methadone 10 mg tablets, 2500 Methadone 20 mg tablets and 2500 Methadone 40 mg tablets.

**Table 10 - Batch 7859 and 7859 tablets characteristics**

| | **Methadone 5mg tab** | **Methadone 10mg tab** | **Methadone 20mg tab** | **Methadone 40mg tab** |
|---|---|---|---|---|
| | **Batch 7859/A** | **Batch 7859/B** | **Batch 7860/A** | **Batch 7860/B** |
| Theoretical tablet weight | 100 mg | 200 mg | 200 mg | 400 mg |
| Tablet tool shape | Round 7.0mm R7 | Round 8.5mm R6.5 | Round 8.5mm R6.5 | Round 9.5mm R9.5 |
| Obtained weight (n=20) | 102 mg | 202 mg | 201 mg | 400 mg |
| Obtained Hardness (n=10) | 57 N | 65 N | 60 N | 60 N |
| Obtained Thickness (n=10) | 3.00 mm | 4.47 mm | 4.41 mm | 6.21 |
| Observed stickiness | n.a. | n.a. | n.a. | n.a. |
| Disintegration test (n=3) | 4 min | 6 min | 3 min | 2 min |
| Empirical disintegration test in 2ml PW on glass/flame | 1' 20" | 1' 30" | 1' 30" | 1' 10" |

Tablets of the invention containing levomethadone and without naloxone can be prepared as above by compensating the lower API amount by mannitol.

### EXAMPLE 3 - Preclinical evaluation of methadone+naloxone 50:1 and levo-methadone+naloxone 25:1 formulations (p.os and i.v.) on the crisis of withdrawal from morphine

### MATERIALS AND METHODS

### Animals

Male Sprague-Dawley rats (Envigo, Varese, Italy) weighing approximately 200-250 g at the beginning of the experimental procedure will be used. Animals will be housed in Ce.S.A.L. (Centro Stabulazione Animali da Laboratorio, University of Florence, Florence, Italy) and used at least one week after their arrival. Four rats will be housed per cage (size 26 × 41 cm2); animals will be fed a standard laboratory diet and tap water ad libitum, and kept at 23 ± 1 °C with a 12 h light/dark cycle, light at 7 a.m. All animal manipulations will be carried out according to the Directive 2010/63/EU of the European Parliament and of the European Union council (22 September 2010) on the protection of animals used for scientific purposes. All efforts will be made to minimize animal suffering and to reduce the number of animals used.

### Oral gavage

The animal's head was hold in place by gently extending the head back-this extension of the head creates a straight line through the neck and esophagus. The gavage needle was placed in the mouth. The needle was then gently advanced along the upper palate until the esophagus is reached. Once proper placement was verified, the material was slowly administered by a syringe attached to the end of the needle. After dosing, the needle was gently removed following the same angle as insertion (Institute for Laboratory Animal Research 2011. Guide for the care and use of laboratory animals, 8th ed Washington (DC): National Academies Press).

### Intravenous administration

The tail was warmed with a lamp for 5 min in order to dilate the vessels. Then a 27G needle was inserted parallel to the tail vein penetrating 2-4 mm into the lumen while keeping the bevel of the needle face upwards. The solution was then injected slowly. No resistance should be felt if the solution is properly administered (Flecknell, P.A. (1987). In Laboratory Animals: An Introduction for New Experimenters (ed. Tuffery), pp. 225-260. John Wiley & Sons, Chichester, England).

### Intraperitoneal administration

Intraperitoneal injections were made in the lower right or left quadrant of the mouse's abdomen. Once the animal was properly restrained, the needle (0.5-inch or less, relatively short to help prevent puncture of the intestines or cecum.) was inserted at approximately a 60° angle to the body wall.

### Treatments

Morphine HCl, methadone HCl (injectable solution 10 mg/1 ml) and naloxone HCl were dissolved in saline solution
(0.9% NaCl) and administered as follow:

### Experimental design

According to Hassan et al. (2020 doi: 10.3389/fpsyt.2020.00411) morphine will be administered following the
scheme below:

| Day | Time (h) | Dose (mg/kg i.p.) |
|---|---|---|
| 1° | 4 p.m | 10 |
| 2° | 9 a.m-4 p.m. | 10 |
| 3° | 9 a.m-4 p.m. | 20 |
| 4° | 9 a.m-4 p.m. | 30 |
| 5° | 9 a.m-4 p.m. | 40 |
| 6° | 9 a.m-4 p.m. | 50 |
| 7° | 9 a.m | 60 |
| 8°-25° | 9. a.m. | daily observation for 60 min of the parameters mentioned below |

### Treatment with methadone or methadone/naloxone

**Table 11 - Starting from day 8 methadone or methadone/naloxone will be administered as follows for each group of treatment, each group of treatment will consist of 10 rats:**

| Pre-treatment | Treatment | |
|---|---|---|
| Days 1-7 | days 8-25 | |
| 1. saline | saline | administration |
| i.p. | p.o. | daily |
| 2. morphine i.p. | saline p.o. | daily administration |
| 3. morphine i.p. | Met 2.5 mg/kg p.o. | daily administration |
| 4. morphine i.p. | Met(2.5 mg/kg)+nal(40:1) p.o. | daily administration |
| 5. morphine i.p. | Met(2.5 mg/kg)+nal(50:1) p.o. | daily administration |
| 6. morphine i.p. | Met(2.5 mg/kg)+nal(60:1) p.o. | daily administration |
| 7. saline i.p. | saline i.v. | daily administration |
| 8. morphine i.p. | Met 2.5 mg/kg i.v. | days: 9,12,15,18,21,24 |
| | Met 2.5 mg/kg p.o. | days: 10-11,13-14,16-17,19-20,22-23,25 |
| 9. morphine i.p. | Met(2.5 mg/kg)+nal(40:1) i.v. | days: 9,12,15,18,21,24 |
| | Met(2.5 mg/kg)+nal(40:1) p.o. | days: 10-11,13-14,16-17,19-20,22-23,25 |
| 10. morphine i.p. | Met(2.5 mg/kg)+nal(50:1) i.v. | days: 9,12,15,18,21,24 |
| | Met(2.5 mg/kg)+nal(50:1) p.o. | days: 10-11,13-14,16-17,19-20,22-23,25 |
| 11. morphine i.p. | Met(2.5 mg/kg)+nal(60:1) i.v. | days: 9,12,15,18,21,24 |
| | Met(2.5 mg/kg)+nal(60:1) p.o. | days: 10-11,13-14,16-17,19-20,22-23,25 |
| 12. Morphine i.p. | levo-Met(1.25 mg/kg)+nal(25:1) i.v. | days: 9,12,15, 18,21 |
| | levo-Met(1.25 mg/kg)+nal (25:1) p.os | days: 10, 11, 13,14,16,17,19,20 |

The dose of methadone was chosen on the base of published evidence (Bobula et al., Pharmacol Rep 2009, doi: 10.1016/s1734-1140(09)70183-5; Hassan et al., Frontiers Psychiatry 2020, doi.org/10.3389/fpsyt.2020.00411).

### Preparation of the solutions to be administered

Methadone 10 mg/ml was diluted in saline solution to give a solution of 0.25 mg/ml of methadone. To 9.9 ml of the methadone solution (0.25 mg/ml) was added 0.1 ml of a naloxone solution, 6.5 mg/10ml, 5 mg/10ml and 4.2 mg/10ml respectively to prepare 10 ml of a solution methadone/naloxone 40:1, 50:1 and 60:1 respectively. Levo-methadone 2.5 mg/ml was diluted in saline solution to give a solution of 0.125 mg/ml of levomethadone. To 9.9 ml of the levo-methadone solution (0.125 mg/ml) was added 0.1 ml of a naloxone solution 2.475 mg/10ml, to prepare 10 ml of a solution levo-methadone/naloxone 50:1.

For all administrations the injected volume was 0.1 ml/10 g of mouse.

The dose of methadone was chosen on the base of published evidence (Bobula et al., Pharmacol Rep 2009, doi: 10.1016/s1734-1140(09)70183-5; Hassan et al., Frontiers Psychiatry 2020, doi.org/10.3389/fpsyt.2020.00411).

### Behavioral parameters observed for the evaluation of the withdrawal syndrome

According to Hassan et al. (2020) doi: 10.3389/fpsyt.2020.00411 and Ruiz et al. Br. J. Pharmacol. (1996) 119, 174-182 the following parameters will be observed every day:
Chewing, head shake, exploring, digging, yawning, teeth chattering, wet dog shake, writhing, diarrhoea, squeaking on touch, eye twitch, ptosis, piloerection. Each of these parameters will have a score of 1.

The evaluations of the behavioral parameters will be carried out daily up to day 25.

### RESULTS

The mice were observed by experimenters who were unaware of the treatment received; each experimenter had the task of observing a single cage and recording all the parameters for 60 minutes at the same time or at 10 am every day until the complete disappearance of the morphine withdrawal crisis.

First of all it was possible to observe that increasing doses of morphine, according to the protocol reported from 10 to 60 mg/kg i.p., were able to induce a significant withdrawal crisis starting from the day following the last administration. These symptoms occurred statistically significantly up to the 22nd day of starting treatment or up to the 15th day after the last dose of morphine. The intensity of the symptoms, which was highest in the first few days, was then progressively reduced. Treatment with methadone 2.5 mg/kg p.o. - 2.5 mg/kg i.v. significantly reduced the symptoms for the duration of the treatment and in this group too the score progressively decreased until the symptoms disappeared. In particular, the values of the scores in these groups for all days of observation are about 50% of that seen in the group treated with morphine alone.

Treatments with methadone 2.5 mg/kg + naloxone 40:1, 50:1, 60:1, p.o. and levo-methadone 1.25 mg + naloxone 50:1 p.o. was able to significantly reduce withdrawal symptoms; the score decreases as the days go by until the symptoms disappear similar to the progressive disappearance of withdrawal symptoms that occurs with morphine alone.

Addition of naloxone regardless of dose was irrelevant in the morphine withdrawal score.

Animals treated with methadone 2.5 mg/kg + naloxone i.v. or levo-methadone 1.25 mg + naloxone 50:1 i.v. on days 9, 12, 15, 18, 21 and 24, unlike what was observed in the same animals on the other days in which they received methadone 2.5 mg/kg + naloxone p.o. reported a drastic increase in scores. The number of scores in these days of treatment with methadone 2.5 mg/kg + naloxone i.v. and levo-methadone 1.25 mg + naloxone 50:1 i.v. it is of equal intensity, if not even higher starting from day 18 compared to the group treated with morphine alone (no statistical significance). On day 21, despite the fact that we are in the presence of an i.v. treatment, no variation is observed because even the group treated with morphine alone lost statistical significance compared to the control group. The groups that received methadone 2.5 mg/kg + naloxone 40:1,50:1 and 60: 1 on the days when the naloxone treatment was done via p.o., they show no difference between them, demonstrating that, as seen previously, the addition of naloxone in the different Met/Nal ratios have no influence on the protection of the withdrawal crisis.

From the preclinical data obtained in the present experiment it emerges that the groups treated with methadone or levo-methadone + naloxone 50:1 show a perfectly analogous behavior in the sense that both, with absolutely comparable efficacy, are capable of drastically reducing the morphine withdrawal crisis after treatment p.os but not after i.v. injection.

From the preclinical data obtained it emerges the protection from the symptoms of the withdrawal crisis when methadone alone and with naloxone are given p.o. to all tested ratios (40:1, 50:1, 60:1).

**Table 12. Withdrawal syndrome evaluation.**

| | Treatment | | | |
|---|---|---|---|---|
| Days | 1 | 2 | 10 | 12 |
| 8 | 3.6±0.2 | 91.4±7.7^{§} | 92.3±9.2 | 95.3±8.9 |
| 9 | 3.8±0.4 | 89.4±9.5^{§} | 85.7±9.2 | 81.1±8.6 |
| 10 | 3.3±0.3 | 81.6±8.4^{§} | 39.7±8.3^ | 34.9±7.5^ |
| 11 | 2.5±0.2 | 75.3±9.1^{§} | 35.4±7.6^ | 33.1±5.4^ |
| 12 | 3.5±0.4 | 66.2±6.9^{§} | 68.3±7.5 | 65.1±7.0 |
| 13 | 2.8±0.6 | 55.9±5.8^{§} | 28.7±6.9^ | 30.4±5.1^ |
| 14 | 3.5±0.7 | 46.3±6.1^{§} | 25.1±6.6^ | 27.7±5.6^ |
| 15 | 4.1±0.4 | 39.5±5.6^{§} | 44.4±5.5 | 42.4±5.0 |
| 16 | 3.5±0.4 | 33.1±6.0^{§} | 20.5±4.9^ | 22.9±3.8^ |
| 17 | 3.6±0.5 | 26.2±5.8^{§} | 16.0±3.9^ | 17.2±3.9^ |
| 18 | 4.1±1.5 | 23.4±4.1^{§} | 24.6±5.5 | 23.1±4.4 |
| 19 | 3.8±1.2 | 13.6±2.3^{§} | 6.2±3.4^ | 5.5±3.2^ |
| 20 | 3.7±1.3 | 9.8.2±3.1^{§} | 5.8±2.4 | 4.3±2.0^ |
| 21 | 3.9±0.2 | 5.4±2.2 | 6.9±6.0 | 5.3± 2.7 |
| 22 | 2.6±0.5 | 3.2±1.3 | 4.5±1.7 | |

The mean score (± S.E.M.; n=10) was evaluated day by day from day 8 to day 22 in the different groups of treatment. ^{§}P<0.05 vs saline + saline (group 1); ^P<0.05 vs morphine + saline

## Claims

1. An immediate release oral solid pharmaceutical formulation consisting of all water-soluble components and comprising methadone (Met) or levomethadone (Lev) and optionally naloxone (Nal) in a weight ratio Met/Nal of 40-60:1, or Lev/Nal of 20-30:1; said solid formulation being free of insoluble critical excipients and totally dissolving in water.

2. The solid formulation according to claim 1 which is a tablet, preferably a swallowable tablet.

3. The solid formulation according to any one of claims 1-2 wherein the critical excipients are microcrystalline cellulose and/or maize starch and/or magnesium stearate.

4. The solid formulation according to any one of claims 1-3 the water-soluble components comprise at least a binder, at least a filler, and at least a lubricant.

5. The solid formulation according to any one of claims 1-4 wherein the at least a binder is selected in the group consisting of lactose, xylitol, maize maltodextrin and mixture thereof.

6. The solid formulation according to any one of claims 1-5 wherein the at least a filler is selected in the group consisting of mannitol.

7. The solid formulation according to any one of claims 1-6 wherein the at least a lubricant is selected in the group consisting of poloxamer.

8. The solid formulation according to any one of claims 1-7 containing 5, 10, 20 or 40 mg of methadone.

9. The solid formulation according to any one of claims 1-8 containing 2.5, 5, 10 or 20 mg of levomethadone.

10. The solid formulation according to any one of claims 1-9 consisting of:
| component | % w/w |
|---|---|
| Methadone HCl | 4.0-10.0 |
| Naloxone HCl | 0.1-0.2 |
| Mannitol | 45-60 |
| Lactose | 30-40 |
| Poloxamer 188 | 3.0-5.0 |
or
| component | % w/w |
|---|---|
| levomethadone HCl | 2.0-5.0 |
| Naloxone HCl | 0.1-0.2 |
| Mannitol | 47-62 |
| Lactose | 30-40 |
| Poloxamer 188 | 3.0-5.0 |
or
| component | % w/w |
|---|---|
| Methadone HCl | 4.0-10.0 |
| Naloxone HCl | 0.1-0.2 |
| Mannitol | 60.0-70.0 |
| Maltodextrin | 7.0-13.0 |
| xylitol | 5.0-10.0 |
| Poloxamer 188 | 7.0-13.0 |
or
| component | % w/w |
|---|---|
| levomethadone HCl | 2.0-5.0 |
| Naloxone HCl | 0.1-0.2 |
| Mannitol | 65.0-75.0 |
| Maltodextrin | 7.0-13.0 |
| xylitol | 5.0-10.0 |
| Poloxamer 188 | 5.0-7.0 |
or
| component | % w/w |
|---|---|
| Methadone HCl | 4.0-10.0 |
| Mannitol | 45-60 |
| Lactose | 30-40 |
| Poloxamer 188 | 3.0-5.0 |
or
| component | % w/w |
|---|---|
| levomethadone HCl | 2.0-5.0 |
| Mannitol | 47-62 |
| Lactose | 30-40 |
| Poloxamer 188 | 3.0-5.0 |
or
| component | % w/w |
|---|---|
| Methadone HCl | 4.0-10.0 |
| Mannitol | 60.0-70.0 |
| Maltodextrin | 7.0-13.0 |
| xylitol | 5.0-10.0 |
| Poloxamer 188 | 7.0-13.0 |
or
| component | % w/w |
|---|---|
| levomethadone HCl | 2.0-5.0 |
| Mannitol | 65.0-75.0 |
| Maltodextrin | 7.0-13.0 |
| xylitol | 5.0-10.0 |
| Poloxamer 188 | 5.0-7.0 |

11. A process of preparing a solid formulation according to any one of claims 1-10, said process comprising:
if naloxone is present serially diluting naloxone in a portion of the filler,
blending all the ingredients except the lubricant,
successively blending the lubricant and then direct compressing.

12. The solid formulation according to any one of claims 1-10 for use in the maintenance treatment of opioid addiction.
